# EUROPEAN PATENT SPECIFICATION

(11) **EP 2 558 585 B1**
(45) Date of publication and mention of the grant of the patent: **17.02.2016**
(21) Application number: 10782052.4
(22) Date of filing: 02.08.2010
(51) Int. Cl.: C12P 7/26, C12P 7/24, C12N 9/88

(54) **A recombinant process for the production of R-aromatic alpha-hydroxy ketones**
Verfahren zur rekombinanten Herstellung von R-aromatischen alpha-Hydroxyketonen
Procédé de production de cétones (R)-alpha-hydroxyliques aromatiques

(30) Priority: 13.04.2010 IN MU12332010
(43) Date of publication of application: 20.02.2013
(73) Proprietor: Indian Institute of Technology, Bombay, Mumbai 400 076 Maharashtra (IN); Embio Limited, Mumbai 400 076 Maharashtra (IN)
(72) Inventor: NORONHA, Santosh, Mumbai 400 076 Maharashtra (IN); AGRAWAL, Praveen, Kumar, 400 076 Jaipur Rajasthan (IN); BHAT, P., Jayadeva, Mumbai 400 076 Maharashtra (IN)
(74) Representative: Eve, Rosemary Margaret
(86) International application number: PCT/IN2010/000511
(87) International publication number: WO 2011/128907

(56) References cited:
- US-A- 5 312 742
- BAYKAL A ET AL: "Synthesis with good enantiomeric excess of both enantiomers of alpha-ketols and acetolactates by two thiamin diphosphate-dependent decarboxylases", BIOORGANIC CHEMISTRY, ACADEMIC PRESS INC., NEW YORK, NY, US, vol. 34, no. 6, 1 December 2006 (2006-12-01), pages 380-393, XP024896869, ISSN: 0045-2068, DOI: 10.1016/J.BIOORG.2006.09.006 [retrieved on 2006-12-01] cited in the application
- IDING H ET AL: "APPLICATION OF ALPHA-KETO ACID DECARBOXYLASES IN BIOTRANSFORMATIONS", BIOCHIMICA ET BIOPHYSICA ACTA. PROTEIN STRUCTURE AND MOLECULAR ENZYMOLOGY, ELSEVIER, AMSTERDAM; NL, vol. 1385, no. 6, 29 June 1998 (1998-06-29), pages 307-322, XP000938920, ISSN: 0167-4838, DOI: 10.1016/S0167-4838(98)00076-4
- STEFAN HOHMANN: "Characterization of PDC6, a third structural gene for pyruvate decarboxylase in Saccharomyces cerevisiae", JOURNAL OF BACTERIOLOGY, AMERICAN SOCIETY FOR MICROBIOLOGY, WASHINGTON, DC; US, vol. 173, no. 24, 1 December 1991 (1991-12-01), pages 7963-7969, XP008124999, ISSN: 0021-9193

## Description

### FIELD OF INVENTION

The present invention relates to a process for the production of (R) - aromatic α-hydroxy ketones of formula (I). In particular, the present invention relates to a process for enhanced production of said (R) - aromatic α-hydroxy ketones of formula (I) in a genetically modified yeast strain having mutation in the regulatory site of Pyruvate decarboxylase.

### BACKGROUND OF THE INVENTION

(R) - phenylacetylcarbinol ((R)-PAC) an optically active compound, is an important aromatic α-hydroxy ketone formed by the acyloin condensation of pyruvic acid with benzaldehyde by the enzyme pyruvate decarboxylase. This enzyme is widely distributed in yeasts, fungi, plants and is also found in some bacteria. (R)-PAC is a precursor and a key intermediate for preparation of compounds such as ephedrine and pseudo-ephedrine, which are two major pharmaceutically active agents found in a wide range of pharmaceutical formulations. These compounds are adrenergic sympathomimetic agents and have anti-histamine activity. Pseudo-ephedrine is useful as a nasal decongestant and is found as an ingredient in cough and cold capsules, sinus medications, nose sprays, nose drops and allergy and hay fever medications. Ephedrine is useful as a topical nasal decongestant, a treatment for mild forms of shock (CNS stimulant) and as a bronchodilator. (R)-PAC has also found application as a chiral auxiliary.

For many decades, (R)-PAC has been obtained by biotransformation of benzaldehyde by pyruvate decarboxylase (PDC) using various species of fermenting yeast. PDCs used in such biotransformation are mainly isolated from *Saccharomyces cerevisiae*, *Kluyveromyces marxianus, Neurospora crassa, Candida utilis,* and *Rhizopus javanicus* (O. P. Ward, A. Singh, Curr. Opin. Biotechnol. 2000, 11, 520;) and the bacterium *Zymomonas mobilis,* (B. Rosche, V. Sandford, M. Breuer, B. Hauer, P. L. Rogers, J. Mol. Catal. B: Enzym. 2002, 19 - 20, 109).

Efforts have been made for *in vitro* production of (R)-PAC by isolating PDC. This process has an advantage of getting product in high purity with negligible formation of by-products. However, these procedures suffer from one or other drawback such as the cost of enzyme production, stability of enzyme, need external addition of pyruvate and cost of immobilization. Consequently, *in vitro* processes are not economically feasible. The whole cell classical biotransformation process itself suffers from several drawbacks, such as formation of by-products (mostly benzyl alcohol) by different reducing systems in the yeast (S. Panke, M. Wubbolts, Curr. Opin. Chem. Biol. 2005, 9, 188), as seen in scheme below.

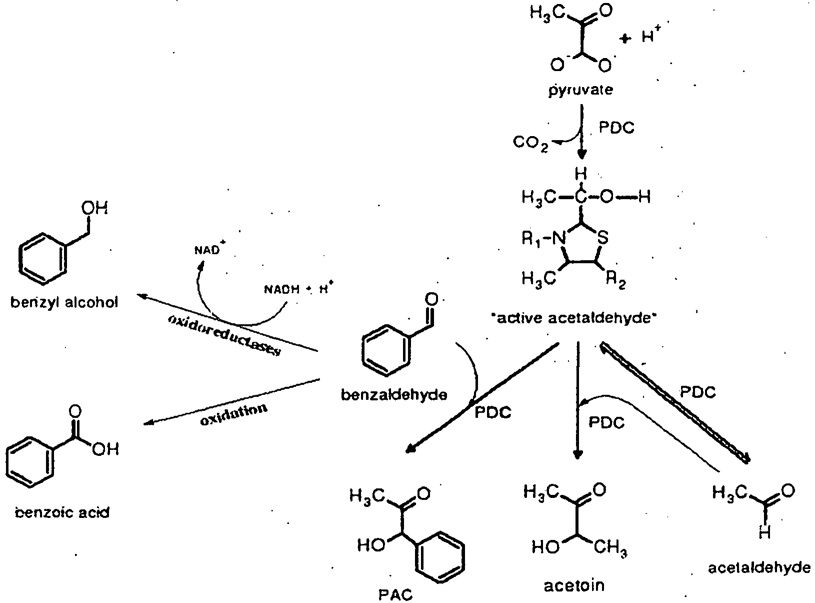

Most of the literatures concerning the synthesis of (R)-PAC by fermenting yeast deal with yield optimization. The available literature suggests that the current yeast transformation of benzaldehyde to (R)-PAC is inefficient and yeast productivity is low. The yeast cannot be used for multiple batches because (R)-PAC production drops with increased exposure to the substrates and to the end product. In addition there exist several reports of enhanced productivity as a consequence of optimization of nutrients, solvent or reactor conditions. There is a general consensus that a high concentration of yeast cells is needed to obtain even relatively low levels of PAC.

US5312742 relates to a process for producing L-PAC by culturing mutants of S. cerevisiae or Candida flareri in the presence of benzaldehyde and pyruvate, whereby PAC in a concentration of at least 12 g/L is formed in the fermentation medium.

Most literature approaches to (R)-PAC process enhancement involve improving PDC levels. PDC activity was found to be rate limiting factor in (R)-PAC production by *S. carlsbergensis* (Vojtisek, V. & Netrval, J. Effect of pyruvate decarboxylase activity and of pyruvate concentration on the production of 1-hydroxy-1-phenylpropanone in Saccharomyces carlsbergensis. Folia Microbiol (Praha), 1982, 27, 173-177), and low PDC concentration was identified as a cause of low R-PAC production in *S. cerevisiae* (Mahmoud, W.; EI-Sayed, A.-H. & Coughlin, R. Biotechnology Bioeng, 1990, 36, 55-63). Various methods for induction/increase of PDC activity have been implemented by fermentation optimization techniques (Tripathi, C. K.; Agarwal, S. C.; Bihari, V.; Joshi, A. K. & Basu, S. K. Production of (L)-phenylacetylcarbinol by free and immobilized yeast cells. Indian J Exp Biol, 1997, 35, 886-889, Culik et al, 1984 Czech Patent No: 222941). However, an increase in PDC activity by overexpression of the enzyme does not necessarily imply a better (R)-PAC process; various groups have over expressed PDC1 *in vivo* towards increasing ethanol levels (a consequence of decarboxylation reaction) and found that the increase in PDC activity *in vivo* does not increase ethanol levels in S. *cerevisiae* (Schaaff I.; Heinisch, J. & Zimmermann, F. K. *Yeast,* 1989, 5, 285-290, and van Hoek, P.; Flikweert, M. T.; van der Aart, Q. J.; Steensma, H. Y.; van Dijken, J. P. & Pronk, J. T. *Appl Environ Microbiol,* 1998, *64*, 2133-214 ). Ethanol levels do increase when PDC is expressed in other organisms with no/less PDC activity (e.g *E. coli* and *Bacillus sp*). In a (R)-PAC production process, increasing PDC would increase both decarboxylation and carboligation activities.

There are further reports in the literature of specific structural modifications of PDC1 (one of the six isoenzymes of PDC) from *S. cerevisiae* and *Z. mobilis.* The enzyme from *Zymomonas* is capable of accepting an acetaldehyde as a substrate (instead of pyruvate) and incorporating it into (R)-PAC. Since acetaldehyde is substantially cheaper than pyruvate, there has been focus on modifying the bacterial enzyme via a site-directed mutagenesis approach. Nevertheless, the conversion efficiencies with the bacterial enzyme are low and consequently the yeast enzyme is currently considered superior for (R)-PAC synthesis, by either *in vivo* or *in vitro* routes. Bruhn *et al* report a modification which influences the carboligation reaction. A mutation is carried out at the active site of ZmPDC, where the tryptophan residue at position 392 is replaced by alanine. Such a mutation influences the decarboxylase/carboligase activity and stability of pyruvate decarboxylase and increases carbotigation activity by four fold and also decreases decarboxylation activity by 2 fold.

ScPDC1 is a homotetramer coded for by a 1692 base pair nucleotide sequence. ScPDC1 exhibits Hill kinetics with respect to pyruvate whereas ZmPDC exhibits Michaelis-Menten type of behavior. Yeast PDC has two pyruvate binding sites: a regulatory site and an active site. The regulatory site must be bound by a pyruvate before the active site takes up a second pyruvate and catalyzes it to acetaldehyde during the (normal) decarboxylation reaction. Thiamine diphosphate and Magnesium dependent PDC catalyzes two types of reactions: decarboxylation (C-C bond breakage), and also carboligation (C-C bond formation). In the PAC process, PDC uses active site bound acetaldehyde ("active aldehyde") obtained by the decarboxylation of the pyruvate, and carboligates it to externally provided benzaldehyde (S. Panke, M. Wubbolts, Curr. Opin. Chem. Biol.. 2005, 9, 188). However, the overall efficiency of the pyruvate utilization for the carboligation reaction remains very low, with a very small proportion of the pyruvate being converted to the desired product, and the bulk of pyruvate undergoing decarboxylation to acetaldehyde.

There are four cysteine residues present in the sequence of PDC1: C69, C152, C221; and C222. C69 is buried deep inside the molecule, whereas the other three are accessible to solvent. Hubser et al., Hubner, G., Konig, S., Schellenberger, A. 1988 Biomedica Biochimica Acta 47 (1), pp. 9-18, Konig, S., Hubner, G., Schellenberger. A. 1990 Biomedica Biochimica Acta 49 (6), pp. 465-471) proposed that the cysteine residues are responsible for the hysteretic substrate activation behaviour of pyruvate decarboxylase.

Based on site directed mutagenesis and steady-state kinetic experiments, Baburina *et. al.* (Baburina, I., Gao, Y., Hu, Z., Hohmann, S., Furey, W., & Jordan,F. (1994) Biochemistry 33, 5630-5635) proposed that C221 is a primary binding site of the regulatory substrate molecule. A substrate analogue study with pyruvamide by Schnieder et. al (Lu, G.; Dobritzsch. D.; Baumann, S.; Schneider, G. & Konig g, S. Eur J Biochem, 2000, 267, 861-868) identified that a nitrogen atom of the side chain of H115 from the O- subunit and the carboxyl group of E477 from the C- subunit are the key catalytic residues. These amino acid residues were already identified as important catalytic residues by site-directed mutagenesis in PDC from both *Z. mobilis* and S. *cerevisiae* (Chang, A. K.; Nixon, P. F. & Duggleby, R. G. Biochem J, 1999, 339 (Pt 2), 255-260, and Schenk, G.; Leeper, F. J.; England, R.; Nixon, P. F. & Duggleby, R. G. Eur J. Biochem, 1997, 248, 63-71)(Duggleby et al. (Baburina, I., Li, H., Bennion, B., Furey, W., & Jordan, F. (1998) Biochemistry 37, 1235-1244.).

Wei *et al.* (Wen Wei, Min Liu, and Frank Jordan; Biochemistry, 2002, 41 (2), pp 451-461) reported work on C221S/C222A and C221E/C222A variants in which a negative charge is built onto the C221 side chain, to better mimic the effect of a pyruvate molecule covalently bonded to C221 as a thiohemiketal. These variants are nearly as active as the wild type enzyme, yet show no substrate activation. Their Results indicate that the effect of C221 substitutions on transition states starts with the binding of the first substrate to the enzyme and terminates with the decarboxylation step. Studies carried out by Wei *et al* are directed towards characterizing the decarboxylation reaction. It has been observed that this specific variant is practically as active as wild type PDC1 and the only difference is in the alleviation of substrate activation. These observations are confined to characterizing the enzyme *in vitro* and not *in vivo.* This is relevant in the sense that several reports exist which describe modifications to an enzyme where *in vitro* activity changes are manifest, but where there is no impact on *in vivo* pathway behavior. Specifically, attempts to modify PDC towards enhancing decarboxylation rates and improving *in vivo* ethanol production in yeast, have failed.

There have also been studies on mutations at the active site which affect the extent of carboligation. Jordan *et al* have created various other mutations to study the PDC1. They have conducted studies on the impact of mutations brought about in the active site on carboligation reactions and have shown that there was improvement in carboligation following mutation of E477Q and D28A, D28N (Sergienko, E. A. & Jordan, F. Biochemistry, 2001, 40, 7369-7381). Subsequently they have also analyzed these mutants for R-PAC formation (Baykal, A.; Chakraborty, S.; Dodoo, A. & Jordan, F. Bioorg Chem, 2006, 34, 380-393). The C221 mutant as suggested by Wei *et al* has lower activity compared to WTPDC1 and therefore it was never a good choice for increasing carboligation.

Also, there have been no *in vivo* studies where wild type or mutant forms of PDC have been expressed to enhance the production of (R)-PAC. The only *in vivo* experimentation reported relates to efforts to increase decarboxylation (ethanol) levels.
Although Bruhn et al teaches increasing the activity of the enzyme by a factor of 4 with regards to carboligation, such teachings cannot be extrapolated, because in *S*. *cerevisiae,* an alanine residue is already present at the same position in the active site as taught in *Bruhn et. al.* Moreover, wild type yeast Pdcs have 20 fold higher carboligation activity when compared to wild type ZmPDC (Bruhn et al.). Therefore ZmPDC, even with a 4 fold higher carboligation as a consequence of an active site mutation, is still not equivalent to yeast Pdcs.

Moreover, Shin et al (Production of L-phenylacetylcarbinol (L-PAC) from benzaldehyde using partially purified pyruvate decarboxylase (PDC). Biotechnol Bioeng. 49, 52-62.) clearly discloses that higher PDC activity is not necessary for higher (R)-PAC production. Rogers et al (Rogers, P. L.; Shin, H. S. & Wang, B. Biotransformation for L-ephedrine production. Adv Biochem Eng Biotechnol, 1997, 56, 33-59) also carried out studies to show that higher PDC activity increased the initial rate of (R)-PAC production but that a higher yield could not be assured.

Accordingly, there exists a need in the art for metabolic engineering approaches towards increasing L-PAC levels *in vivo* by modifying PDC such that the carboligation reaction is selectively preferred over the decarboxylation reaction.

Surprisingly, it has now been found that a PDC with improved synthesis capacity with respect to the formation of (R)-PAC can be obtained which has a relatively high selectivity for the carboligation reaction as compared to the decarboxylation reaction, thus increasing the production of (R) - aromatic α-hydroxy ketones in a genetically modified strain of *S*. *cerevisiae,* with PDC mutated at its regulatory site.

The present inventors have found that accumulation of higher levels of (R) aromatic α-hydroxy ketones in a mutated *S. cerevisiae* strain expressing a C221 E/C222A variant of ScPDC1 overcomes drawback found in the prior art.

### OBJECTS OF THE INVENTION

It is an object of the present invention to provide a novel process for enhanced production of (R) - aromatic α-hydroxy ketones.

It is another object of the present invention to provide a process for enhanced production of (R) - aromatic α-hydroxy ketones in genetically modified strain of yeast, preferably *S. cerevisiae,* having a polypeptide that possesses pyruvate decarboxylase activity.

It is yet another object of the present invention to provide a process for enhanced production of (R) - aromatic a-hydroxy ketones in genetically modified strain of yeast, preferably *S. cerevisiae,* having mutations at positions 221 and 222 in the regulatory site of the pyruvate decarboxylase in order to favour the carboligation reaction over decarboxylation.

It is another object of the present invention to provide a process for enhanced biotransformation to (R)- aromatic a-hydroxy ketones by overexpressing any functional PDC in any yeast species, preferably *S. cerevisiae.*

It is another object of the present invention to provide a process for enhanced biotransformation to (R)- aromatic a-hydroxy ketones by overexpressing ScPDC (1, 5 and 6).

It is another object of the present invention to provide a process for enhanced biotransformation to (R)- aromatic a-hydroxy ketones by overexpressing any PDC with mutation at the regulatory site (to remove substrate activation).

### SUMMARY OF THE INVENTION

An aspect of the present invention is to provide a process for preparation of (R)-aromatic α-hydroxy ketones of formula (I), 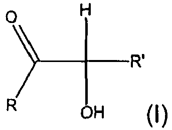 said process occurring in strain(s) of yeast expressing a recombinant pyruvate decarboxylase, having cysteine residues at positions 221 and 222 of PDC1 of *Saccharomyces cerevisiae* (scPDC1), an isoenzyme of said pyruvate decarboxylase, substituted with glutamate and alanine, respectively, such that the said mutation being in the regulatory site of pyruvate decarboxylase, selectively favours carboligation reaction over decarboxylation, wherein R is H or CH₃ and wherein R' is phenyl or optionally substituted phenyl, wherein the substituents are selected from the group C₁₋₃ alkyl, C₁₋₃ alkoxy, F, Cl, Br, I, OH, NH₂, CN and NR₁R₂, wherein R₁, and R₂ can be independently H or (C₁₋₄) alkyl and the said C₁₋₃ alkyl can be further substituted by a substituent chosen from F, Cl, Br, I and OH and also pyridine, thiophene, furan, napthaldehyde and their substituents.

Another aspect of the present invention is to provide a process for preparation of (R)-aromatic α-hydroxy ketones of above formula (I) with R and R' as defined above, said process occurring in strain(s) of yeast expressing a recombinant pyruvate decarboxylase having the cysteine residue at position 221 of PDC5 of *S. cerevisiae* (scPDC5), an isoenzyme of said pyruvate decarboxylase, substituted with glutamate, such that said mutation, being in the regulatory site of pyruvate decarboxylase, selectively favours carboligation reaction over decarboxylation.

Yet another aspect of the present invention is to provide a process for preparation of (R)- aromatic α-hydroxy ketones of above formula (I) with R and R' as defined above, said process occurring in strain(s) of yeast expressing a recombinant pyruvate decarboxylase having the cysteine residue at position 221 of PDC6 of *S. cerevisiae* (scPDC6), an isoenzyme of said pyruvate decarboxylase, substituted with glutamate, such that said mutation, being in the regulatory site of pyruvate decarboxylase, selectively favours carboligation reaction over decarboxylation.

### BRIEF DESCRIPTION OF FIGURES

- Figure 1: A comparison of in *vivo* ethanol formation in wild type strains of *S. cerevisiase* overexpressing PDC1 (BY-P1, open circles) and vector control pGPD (BY-GPO, closed circles).
- Figure 2: *In vitro* comparison of decarboxylation rates, PDC1 (close circle) and PDC1-C221E/C222A (open circle).
- Figure 3: *In vitro* comparison of R-PAC formation rates, PDC1 (close circle) and PDC1-C221E/C222A (open circles).
- Figure 4: Carboligation activity measured in cell free extract of Wild type control strain (8-G) and PDC1 over expressed strain (B-P1)
- Figure 5: Decarboxylation activity measured in cell free extract of Wild type control strain (B-G) and PDC1 over expressed strain (B-P1)
- Figure 6: (R)-PAC production in a wild type laboratory strain (8) of *S. cerevisiae*
- by PDC1 (B-P1)
- Figure 7: (R)-PAC production in a wild type laboratory strain (B) of *S. cerevisiae*
- by PDC5 (B-P5)
- Figure 8: (R)-PAC production in a wild type laboratory strain (B) of *S. cerevisiae*
- by PDC6 (B-P6)
- Figure 9: (R)-PAC production in a wild type laboratory strain (B) of *S. cerevisiae*
- by PDC1 double mutant (B-PD)

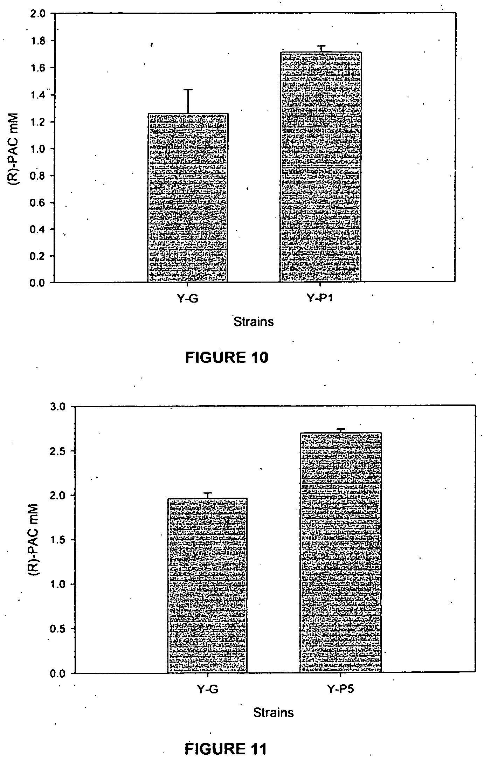

- Figure 10: (R)-PAC production in a wild type laboratory strain (Y) of *S. cerevisiae*
- by PDC1 (Y-P1).
- Figure 11: (R)-PAC production in a wild type laboratory strain (Y) of *S. cerevisiae*
- by PDC5 (Y-P5).

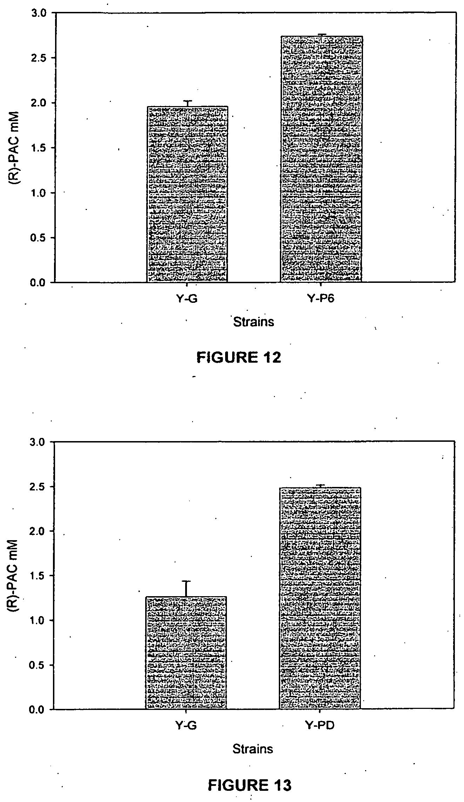

- Figure 12: (R)-PAC production in a wild type laboratory strain (Y) of *S. cerevisiae* by PDC6 (Y-P6)
- Figure 13: (R)-PAC production in a wild type laboratory strain (Y) of *S. cerevisiae* by PDC1 double mutant (Y-PD)
- Figure 14: (R)-PAC production in a pdc null strain (D) of *S. cerevisiae* by PDC1 (P1), PDC5 (P5), PDC6 (P6) and PDC1 double mutant (PD)
- Figure 15: Production of (R) - aromatic α-hydroxy ketones of formula (I) by employing acetaldehyde which is not achievable by employing PDCs from wild type(WT) yeast.

### DETAILED DESCRIPTION OF THE INVENTION

The present invention relates to a method according to the claims for enhancing (R)- aromatic α-hydroxy ketones of formula (I) productivity by way of a recombinant (mutant variant of) *S. cerevisiae* wherein the gene of the key enzyme involved in the biotransformation (Pyruvate decarboxylase) is modified in *S. cerevisiae.* The (R) - aromatic α-hydroxy ketones in the present invention is preferably (R)-phenylacetylcarbinol ((R)-PAC).

In a (R)-PAC production process, increasing PDC would increase both decarboxylation and carboligation activities. It is known that PDC is not rate limiting for decarboxylation (ethanol level does not change) but according to the present inventors it is limiting for carboligation (R-PAC) purposes. Since carboligation requires additional mechanism steps, the decarboxylation reaction may be expected to be several times faster than carboligation.

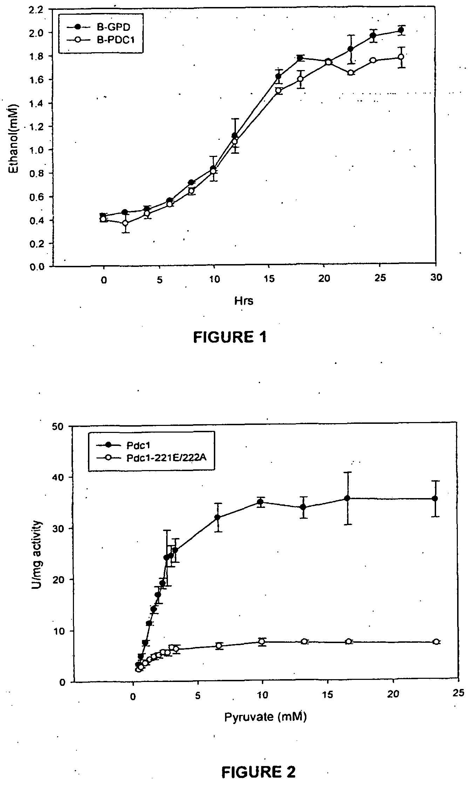

Specifically, attempts to modify PDC towards enhancing decarboxylation rates and improving *in vivo* ethanol production in yeast, have failed and the present inventors have also verified the same the result of which is illustrated in Figure 1. Rate of pyruvate utilization by wild-type and mutant PDC1. Equal amount of purified PDCs were assayed for decarboxylation activity *in vitro.* The rate of pyruvate utilization was calculated for both enzymes and compared. As evident form Figure 2, the mutant has a lowered decarboxylation rate.

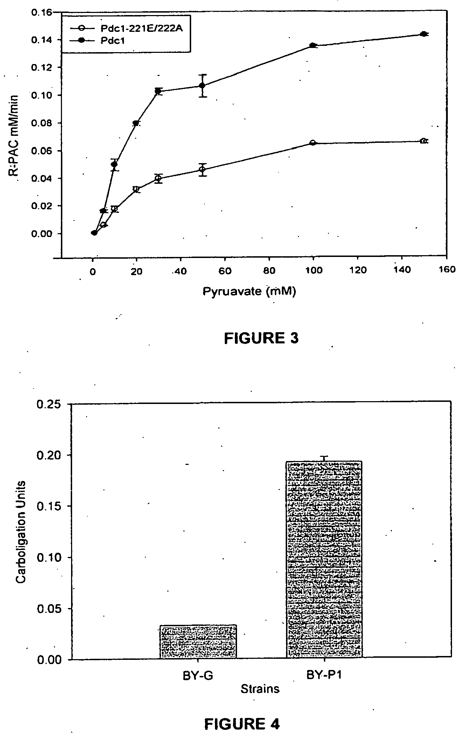

It has also been verified by the present inventors that the C221 E mutation does not increase carboligation activity in *in vitro* conditions. Figure 3 illustrates a comparative graph between a wild type *S. cerevisiae* and mutant ScPDC1. Equal amounts of both proteins were assayed for R-PAC formation against a fixed concentration of benzaldehyde (100mM) and varying concentration of pyruvate (0 to 150 mM). Mutant PDC1 has lower R-PAC formation rate *in vitro.*

The table below provides a comparison of catalytic efficiencies of PDCs for decarboxylation and carboligation from Figures 2 and 3.

| PDC | Vmax (decarboxylation) µmol/min/mg | Vmax (carboligation) mM R-PAC/min |
|---|---|---|
| PDC1 | 35.3 | 0.14 |
| PDC-C221 E/C222A | 7.6 | 0.07 |

It is evident from the above table that a 4 fold decrease was observed in the decarboxylation reaction rate while carboligation rate has decreased only 2 fold. This mutation therefore selectively favors carboligation over decarboxylation.

Six genes for PDC have been identified in *S. cerevisiae*, of which three (Pdc1 5, and 6) are catalytically active and the rest have role in regulation of PDC activity. These three active isoenzymes can be prepared by the known gene sequence from the database by recombinant methods. *S. cerevisiae* genome sequence is known and is available at the yeast genome database (www.yeastgenome.org). Information about the desired genes can be obtained from this database. Based on sequences, specific primers can be designed to amplify the respective genes. (Molecular cloning, Maniatis 1989)

Mutations may lead to conformational changes in the enzyme. But all conformational changes brought about by mutations are not similar and they may produce different effects. Some mutation affects decarboxylation while others may change the carboligation reaction C221E mutant when studied *in vitro* does not favor carboligation, but when expressed *in vivo* results in higher R-PAC production. According to the inventors this could be because of increased intracellular pyruvate levels. This enzyme has decreased decarboxylation activity *in vitro* and this likely reduces pyruvate utilization *in vivo.* Consequently an elevated level of pyruvate likely exists which benefits the progress of the carboligation reaction (which, at least *in vitro,* is affected less by the mutation). Another possible aspect is that the decarboxylated pyruvate may be less easily given off by the mutant, facilitating a condensation with the exogenously added benzaldehyde).

Isolation procedure for the target gene (primers used for amplification of the gene) and characterization of gene was carried out according to the procedure available on vwvw.yeastgenome.org.

The PCR amplification, construction of vectors and transformation of PDC1, PDC5 and PDC6 were carried out according to the standard PCR protocol available at Molecular Cloning by E.F. Fritsch, J. Sambrook, T. Maniatis 1989.

PDC1, PDC5 and PDC6 were modified at substrate regulatory site (Cys221) by site-directed mutagenesis. Single cysteine (Cys221) or both cysteine (Cys221 and Cys222) were replaced with Glu221 or Glu221/Ala222. Mutation of C221E and C221A were done similar to Wei et al., using standard protocol for Site-directed mutagenesis (Quikchange^{™} Site directed mutagenesis protocol adapted from Stratagene).

Further, it has been observed that expression of these modified enzymes in a Pdc null strain produced even higher R-PAC than that obtained by over expression of single native Pdc.

The invention is now demonstrated by way of illustrative examples.

### Example 1

To establish the concept of improved biotransformation by changing PDC activity, these enzymes were expressed in yeast strains grown on a medium selected from one of the following- minimal (defined media), YPD (yeast extract, peptone, dextrose) and a commercial feed stock like molasses. Plasmid for expression of PDC1, PDC5, PDC6 and PDC-221/222 were transformed into various yeast strains.

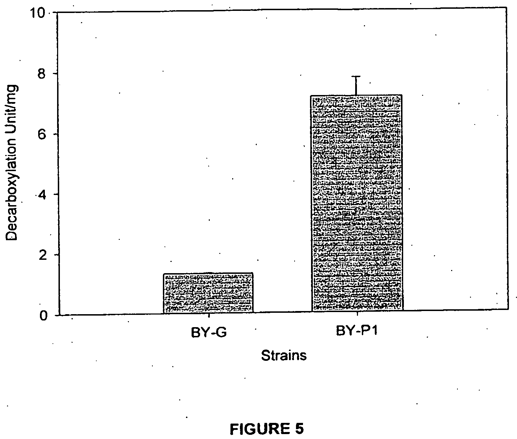

First, enhancement of PDC activity was confirmed by estimation of total activity in cell free extract of recombinant strains. Both decarboxylation and carboligation activities were measured in cell free extract. Figures 4 and 5 illustrate the carboligation and decarboxylation activity measured in cell free extract of Wild type (B-GPD) and PDC1 over expressed strain (B-PDC1) respectively.

After confirmation of increased PDC activity these strains were tested for biotransformation. Biotransformation experiments were conducted at lab scale under controlled .conditions and were compared to results with the parent strain. Over-expression of these enzymes leads to increase in (R) - aromatic α-hydroxy ketones of formula (I) production compared to the parent (non-recombinant) strain. This increase in (R)- aromatic α-hydroxy ketones of formula (I) production was from 10% to 100% with different enzymes, the highest being with the PDC-C221 E/C222A mutant. Effect of PDC activity on biotransformation by these enzymes was determined in various strains.

### Example 2

A wild type strain (B) of *S. cerevisiae* was transformed with.plasmid expressing PDC1, PDC5, PDC6 and PDC1 mutant.

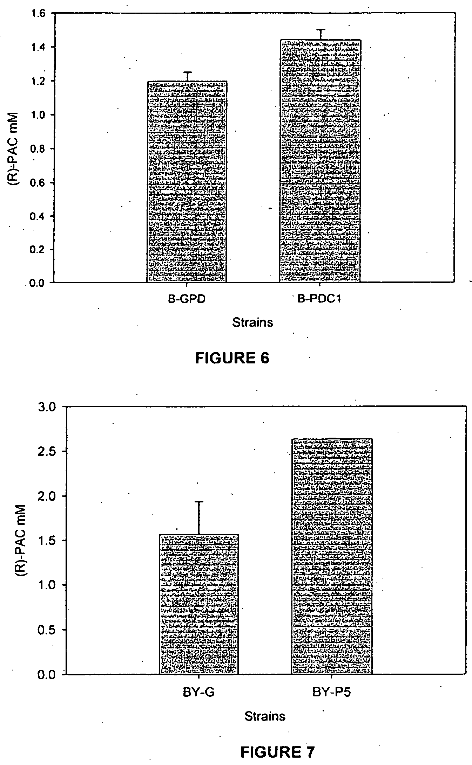

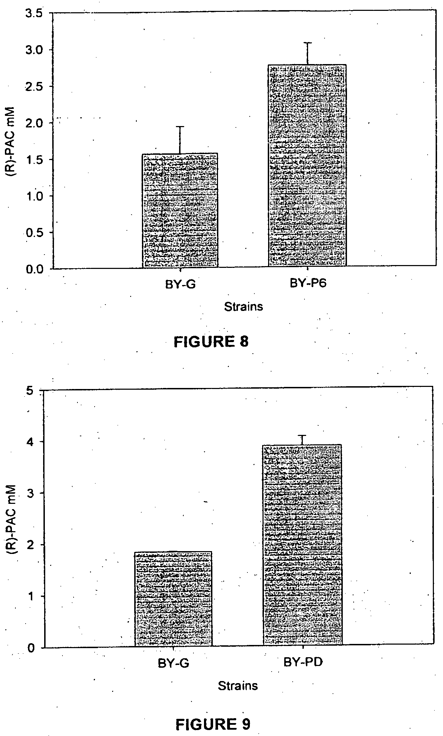

Figures 6 to 9 describe increase in the (R)-PAC by over expression of different PDC enzymes and PDC1 mutant.

### Example 3

To establish that the effect of PDC activity is strain independent, these enzymes were also expressed in another wild-type strain (Y) of *S. cerevisiae.*

Figure: 10 to 13 describe similar increases in the levels of (R)-PAC by over expression of different PDC enzymes and the PDC1 mutant in this alternate host.

### Example 4

Examples 1, 2 and 3 indicated the benefit of increaseing PDC activity in cells. To eliminate the effect of genomic PDC and to study the contribution of recombinant/modified PDC on biotransformation, a strain of *S. cerevisiae* with reduced/null PDC activity was selected for the experiments. Expression of these enzymes in this strain leads to very high production of (R)-PAC.

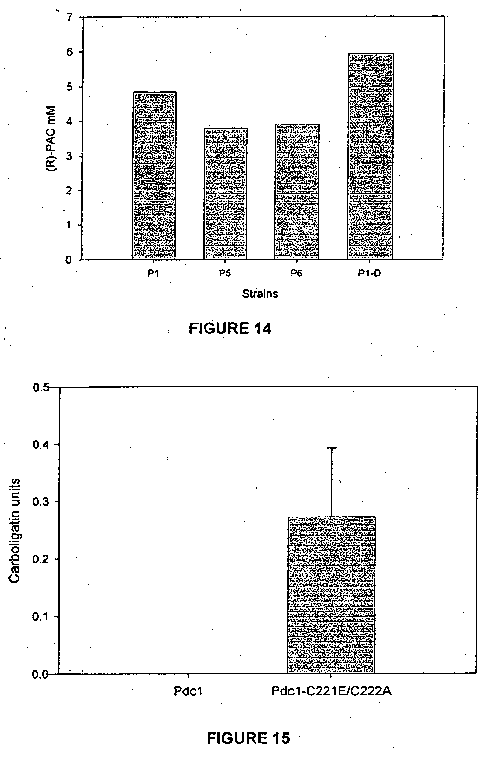

It was observed that the (R)-PAC production was high in all the strains expressing the mutant form of PDC1; and was highest when this form was expressed in the strain with reduces/null activity (Figure 14).

The present invention by way of over-expression of ScPDCs provide the following advantages:
- Improves biotransformation in all *S. cerevisiae* strains. Site directed mutagenesis results in further improvement in the process.
- Production of (R) - aromatic α-hydroxy ketones of formula (I) by employing acetaldehyde which is not achievable by employing the wild type(WT) PDCs of wild type(WT) yeast as illustrated in figure 15.

## Claims

1. A process for preparation of (R)- aromatic α-hydroxy ketones of formula (I), 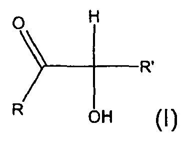 said process occurring in strain(s) of yeast expressing a recombinant pyruvate decarboxylase, having cysteine residues at positions 221 and 222 of PDC1 of *Saccharomyces cerevisiae* (scPDC1), an isoenzyme of said pyruvate decarboxylase, substituted with glutamate and alanine, respectively, such that the said mutation being in the regulatory site of pyruvate decarboxylase, selectively favours carboligation reaction over decarboxylation, wherein R is H or CH₃ and wherein R' is phenyl or optionally substituted phenyl, wherein the substituents are selected from the group C₁₋₃ alkyl, C₁₋₃ alkoxy, F, Cl, Br, I, OH, NH₂, CN and NR₁R₂, wherein R₁, and R₂ can be independently H or (C₁₋₄) alkyl and the said C₁₋₃ alkyl can be further substituted by a substituent chosen from F, Cl, Br, I and OH and also pyridine, thiophene, furan, napthaldehyde and their substituents.

2. The process as claimed in claim 1, comprising isolating a polypeptide encoded by the nucleic acid sequence of scPDC1 from yeasts and substituting the amino acid at the substrate regulatory site at positions 221 and 222.

3. A process for preparation of (R)- aromatic α-hydroxy ketones of above formula (I) with R and R' as defined above, said process occurring in strain(s) of yeast expressing a recombinant pyruvate decarboxylase having the cysteine residue at position 221 of PDC5 of *S. cerevisiae* (scPDC5), an isoenzyme of said pyruvate decarboxylase, substituted with glutamate, such that said mutation, being in the regulatory site of pyruvate decarboxylase, selectively favours carboligation reaction over decarboxylation.

4. A process for preparation of (R)- aromatic α-hydroxy ketones of above formula (I) with R and R' as defined above, said process occurring in strain(s) of yeast expressing a recombinant pyruvate decarboxylase having the cysteine residue at position 221 of PDC6 of *S. cerevisiae* (scPDC6), an isoenzyme of said pyruvate decarboxylase, substituted with glutamate, such that said mutation, being in the regulatory site of pyruvate decarboxylase, selectively favours carboligation reaction over decarboxylation.

5. The process as claimed in claims 1, 3 or 4, wherein the recombinant PDC is introduced in a wild type strain of yeast species selected from *Saccharomyces sp, Kluyveromyces sp, Pichia sp,* Hansenula *sp*.

6. The process as claimed in claims 1, 3 or 4, wherein the recombinant PDC is introduced in a native or recombinant strain of yeast selected from *Saccharomyces sp, Kluyveromyces sp, Pichia sp,* Hansenula *sp*, with reduced/null Pdc activity.

7. The process for preparation of (R)- aromatic α-hydroxy ketones of formula (I) as claimed in claims 1 to 4 wherein the (R)- aromatic α-hydroxy ketone is (R)-phenylacetylcarbinol ((R)-PAC).

## Patentansprüche

1. Verfahren zur Herstellung von (R)- aromatischen α-Hydroxyketonen der Formel (I), 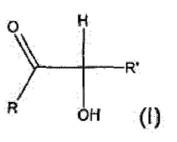 wobei das Verfahren in einem Hefestamm (Hefestämmen) stattfindet, der (die) eine rekombinante Pyruvatdecarboxylase exprimiert (exprimieren), bei der Cysteinreste an den Positionen 221 und 222 von PDC1 von *Saccharomyces cerevisiae* (scPDC1), einem Isoenzym der Pyruvatdecarboxylase, durch Glutamat bzw. Alanin substituiert sind, so dass die besagte Mutation, die sich in der regulatorischen Stelle von Pyruvatdecarboxylase befindet, selektiv eine Carboligationsreaktion gegenüber einer Decarboxylierung begünstigt, wobei R H oder CH₃ ist und wobei R' Phenyl oder gegebenenfalls substituiertes Phenyl ist, wobei die Substituenten ausgewählt sind aus der Gruppe C₁₋₃-Alkyl, C₁₋₃-Alkoxy, F, Cl, Br, I, OH, NH₂, CN und NR₁R₂, wobei R₁ und R₂ unabhängig H oder (C₁₋₄)-Alkyl sein können und das besagte C₁₋₃-Alkyl weiterhin substituiert sein kann durch einen Substituenten ausgewählt aus F, Cl, Br, I und OH und auch Pyridin, Thiophen, Furan, Naphthaldehyd und ihren Substituenten.

2. Verfahren wie in Anspruch 1 beansprucht, umfassend das Isolieren eines durch die Nucleinsäuresequenz von scPDC1 codierten Polypeptids aus Hefen und Substituieren der Aminosäure an der substrat-regulatorischen Stelle an den Positionen 221 und 222.

3. Verfahren zur Herstellung von (R)- aromatischen α-Hydroxyketonen der vorstehenden Formel (I) mit R und R' wie vorstehend definiert, wobei das Verfahren in einem Hefestamm (Hefestämmen) stattfindet, der (die) eine rekombinante Pyruvatdecarboxylase exprimiert (exprimieren), bei der der Cysteinrest an Position 221 von PDC5 von *S. cerevisiae* (scPDC5), einem Isoenzym der Pyruvatdecarboxylase, durch Glutamat substituiert ist, so dass die besagte Mutation, die sich in der regulatorischen Stelle von Pyruvatdecarboxylase befindet, selektiv eine Carboligationsreaktion gegenüber einer Decarboxylierung begünstigt.

4. Verfahren zur Herstellung von (R)- aromatischen α-Hydroxyketonen der vorstehenden Formel (I) mit R und R' wie vorstehend definiert, wobei das Verfahren in einem Hefestamm (Hefestämmen) stattfindet, der (die) eine rekombinante Pyruvatdecarboxylase exprimiert (exprimieren), bei der der Cysteinrest an Position 221 von PDC6 von *S. cerevisiae* (scPDC6), einem Isoenzym der Pyruvatdecarboxylase, durch Glutamat substituiert ist, so dass die besagte Mutation, die sich in der regulatorischen Stelle von Pyruvatdecarboxylase befindet, selektiv eine Carboligationsreaktion gegenüber einer Decarboxylierung begünstigt.

5. Verfahren wie in den Ansprüchen 1, 3 oder 4 beansprucht, wobei die rekombinante PDC in einen Wildtypstamm einer Hefeart, ausgewählt aus *Saccharomyces sp, Kluyveromyces sp, Pichia sp, Hansenula sp,* eingeführt wird.

6. Verfahren wie in den Ansprüchen 1, 3 oder 4 beansprucht, wobei die rekombinante PDC in einen nativen oder rekombinanten Hefestamm, ausgewählt aus *Saccharomyces sp, Kluyveromyces sp, Pichia sp, Hansenula sp,* mit reduzierter/nicht vorhandener PDC-Aktivität eingeführt wird.

7. Verfahren zur Herstellung von (R)- aromatischen α-Hydroxyketonen der Formel (I) wie in den Ansprüchen 1 bis 4 beansprucht, wobei das (R)- aromatische α-Hydroxyketon (R)-Phenylacetylcarbinol ((R)-PAC) ist.

## Revendications

1. Procédé de préparation de (R)-α-hydroxycétones aromatiques de formule(I), 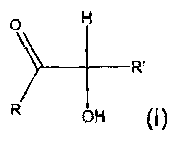 ledit procédé se produisant dans une (des) souche(s) de levure exprimant une pyruvate décarboxylase recombinante, ayant les résidus cystéine aux positions 221 et 222 de la PDC1 de *Saccharomyces cerevisiae* (scPDC1), une isoenzyme de ladite pyruvate décarboxylase, substituées par un glutamate et une alanine, respectivement, de sorte que ladite mutation étant dans le site régulateur de la pyruvate décarboxylase, favorise sélectivement la réaction de carboligature par rapport à la décarboxylation, dans lequel R représente H ou CH₃ et dans laquelle R' est un phényle ou un phényle éventuellement substitué, dans lequel les substituants sont sélectionnés parmi les groupes alkyle en C₁₋₃, alcoxy en C₁₋₃, F, Cl, Br, I, OH, NH₂, CN et NR₁R₂, où R₁ et R₂ peuvent être indépendamment H ou alkyle (en C₁₋₄) et ledit alkyle en C₁₋₃ peut encore être substitué par un substituant choisi parmi F, Cl, Br, I et OH et également une pyridine, un thiophène, un furane, un naphtaldéhyde et leurs substituants.

2. Procédé selon la revendication 1, comprenant l'isolement d'un polypeptide codé par la séquence d'acide nucléique de scPDC1 provenant de levures et la substitution de l'acide aminé au niveau du site régulateur du substrat aux positions 221 et 222.

3. Procédé de préparation de (R)-α-hydroxycétones aromatiques de formule (I) ci-dessus, R et R' étant tels que définis ci-dessus, ledit procédé se produisant dans une (des) souche(s) de levure exprimant une pyruvate décarboxylase recombinante ayant le résidu cystéine à la position 221 de la PDC5 de *S. cerevisiae* (scPDC5), une isoenzyme de ladite pyruvate décarboxylase, substituée par un glutamate, de sorte que ladite mutation, étant dans le site régulateur de la pyruvate décarboxylase, favorise sélectivement la réaction de carboligature par rapport à la décarboxylation.

4. Procédé de préparation de (R)-α-hydroxycétones aromatiques de formule (I) ci-dessus R et R' étant tels que définis ci-dessus, ledit procédé se produisant dans une (des) souche(s) de levure exprimant une pyruvate décarboxylase recombinante ayant le résidu cystéine à la position 221 de la PDC6 de *S. cerevisiae* (scPDC6), une isoenzyme de ladite pyruvate décarboxylase, substituée par un glutamate, de sorte que ladite mutation étant dans le site régulateur de la pyruvate décarboxylase, favorise sélectivement la réaction de carboligature par rapport à la décarboxylation.

5. Procédé selon les revendications 1, 3 ou 4, dans lequel la PDC recombinante est introduite dans une souche de type sauvage d'espèce de levure sélectionnée parmi *Saccharomyces sp, Kluyveromyces sp, Pichia sp, Hansenula sp*.

6. Procédé selon les revendications 1, 3 ou 4, dans lequel la PDC recombinante est introduite dans une souche native ou recombinante de levure sélectionnée parmi *Saccharomyces sp, Kluyveromyces sp, Pichia sp, Hansenula sp,* ayant une activité Pdc réduite/nulle.

7. Procédé de préparation de (R)-α-hydroxycétones aromatiques de formule (I) selon les revendications 1 à 4, dans lequel la (R)-α-hydroxycétone aromatique est le (R)-phénylacétylcarbinol ((R)-PAC).
